# EUROPEAN PATENT APPLICATION

(11) **EP 3 085 702 A1**
(43) Date of publication of application: **26.10.2016**
(21) Application number: 14871834.9
(22) Date of filing: 17.01.2014
(51) Int. Cl.: C07H 13/06, A61K 31/7024

(54) **BIOCONJUGATE MOLECULES WITH BIOLOGICAL AND TECHNO-FUNCTIONAL ACTIVITY, METHOD FOR THE PRODUCTION THEREOF AND USE THEREOF**

(30) Priority: 18.12.2013 MX 2013015020
(71) Applicant: Centro De Investigación Y Asistencia En Tecnología Y Diseno Del Estado De Jalisco, A.C. (CIATEJ, A.C.), 44270 Guadalajara, Jalisco (MX); Kurago Biotek Holdings S.A.P.I. De C.V., 45050 Zapopan, Jalisco (MX)
(72) Inventor: SANDOVAL FABIÁN, Georgina Coral, 44260 Guadalajara, Jalisco (MX); ARRIZON GAVIÑO, Javier Plácido, 44300 Guadalajara, Jalisco (MX); GONZÁLEZ ÁVILA, Marisela, 44820 Guadalajara, Jalisco (MX); PADILLA CAMBEROS, Eduardo, 45184 Zapopan, Jalisco (MX); MARTÍNEZ VELÁZQUEZ, Moisés, 45130 Zapopan, Jalisco (MX); VILLANUEVA RODRÍGUEZ, Socorro Josefina, 53378 Naucalpan de Juárez (MX); CASAS GODOY, Leticia, 45060 Zapopan, Jalisco (MX)
(74) Representative: Jeannet, Olivier
(86) International application number: PCT/MX2014/000013
(87) International publication number: WO 2015/093929

(57) **Abstract**

The present invention regards the synthesis of bioconjugated molecules, formed between two or more of the following functional groups: sugars, prebiotics, oligosaccharides, polysaccharides, triglycerides, fatty acids, fatty acids esters, anti-inflammatories; with its production process by biocatalyzed synthesis with hydrolases such as esterases, proteases, lipases or cutinases, and its purification with several methods that include washing and drying. In addition, its applications in foods, pharmaceuticals and cosmetics, such as: prebiotic nutraceutical, anti-inflammatory, antitumoral, intestinal vector, techno-functional ingredient for food applications (emulsifier, fat substitute) and cosmetic emollient; which are possible since these are non toxic molecules according to the Ames tests.

## Description

### FIELD OF THE INVENTION

The present invention has its technical field in the area of biotechnology since it produces bioconjugated molecules, formed between two or more of the following functional groups: sugars, prebiotics, oligosaccharides, polysaccharides, triglycerides, fatty acids, fatty acids esters, anti-inflammatories; with its production process (synthesis and purification); and its applications as prebiotic nutraceutical, anti-inflammatory, antitumoral, intestinal vector, techno-functional ingredient for food applications (emulsifier, fat substitute) and cosmetic; which are possible since these are non toxic molecules. They will be used for human and veterinarian applications.

### BACKGROUND

Carbohydrate fatty acid sugars esters (CFAE) are chemically classified as non ionic surfactants with a carbohydrate hydrophilic head with one ore more fatty acids as lipophilic component, with biological and techno functional properties of interest. The main properties of these bioconjugates, in comparison with other typical surfactants obtained form petroleum, are their biodegradability and absence of toxicity, besides they can be produced form removable natural sources like fatty acids and carbohydrates (Allen and Tao, 1999). Therefore they are usually used as surfactant and emulsifiers in pharmaceutical, cosmetic and food industries (Chang and Shaw, 2009). CFAE can be synthetized chemically or enzymatically (Plat and Linhardt, 2001).

In the first case, the traditional method for saccharose esters synthesis is based in the transesterification between a fatty acid methyl or ethyl ester and the disaccharides, in an aprotic solvent using basic catalysis (potassium carbonate) at high temperatures and pressure. Depending on the amount of acylating agent and the reaction time, sucrose esters with different degrees of substitution are obtained. The reaction generates a mixture of regio-isomers and the production yield is below 50% (Osipow et al., 1956). This is the process usually used industrially. Besides the low yield obtained, it produces colored sub products, and for its application in the food industry all toxic solvents must be removed (this is not easy due to the high boiling points they have).

For the second case, enzymatic acylation of carbohydrates (specially mono- and disaccharides) is catalyzed by hydrolases with a technique that although is not new, it presents challenges such as selection of solvents, enzymes and enzymatic support (Plou et al., 2002). The main advantage of enzymatic acylation of carbohydrates regarding chemical synthesis is its high regioselectivity that avoids using long processes of protecting and unprotecting. With enzymatic bioconjugation no soaps are formed. In addition, the reaction conditions are mild, while in direct chemical acylation extreme conditions are used (e.g. temperatures over 100°C that can caramelize sugars). From the "marketing" point of view there is another advantage, CFAE produced enzymatically can be labeled as "natural" surfactants (Sarney and Vulfson, 1995).

Until now enzymatic esterification of simple sugars such as glucose (Ruela et al., 2013), short fructo-oligo-saccharides (FOS) (Sagis et al., 2008; ter Haar et al., 2010) and starch (Alissandratos et al., 2010) have been reported. However, unlike the present invention, the FOS used in the previously mentioned works, use lineal fructans with β(2→1) links. Before the present invention, no previous reports of enzymatic esterification of branched fructans like the ones of *Agave tequilana,* with β(2→1) y β(2→6) links (Lopez et al., 2003; Mellado-Mojica and López, 2012; Praznik et al., 2013), were found. The presence of this type of links will confer different properties to FOS. For example, they would be more hydrophilic than lineal FOS like inulin and would have benefits in intestinal health at a prebiotic level (Gomez et al., 2010). Another novelty of this invention is that no previous reports were found where oils, esters or omega-3 fatty acids were used for the acylation of sugars to produced bioconjugates. Therefore, the enzymatic bioconjugation of fructans form *A. tequilana* with different fatty acids, including omega-3, represent an opportunity for innovation. In addition, this represents a scientific challenge caused by the importance of the regioselectivity against the different OH of the prebiotics, that makes biocatalysis a powerful tool that takes advantage of enzyme selectivity in innocuous and environmentally friendly conditions.

For these reactions, activity and specificity of hydrolases, and the characteristics of the products obtained, are influenced by the nature of the organic solvent. The optimal reaction conditions compromise maximal enzymatic activity and substrate solubility. The problem increases with the size of the sugar: monosaccharide < disaccharide < trisaccharide < oligosaccharide < polysaccharide.

Several strategies exist to overcome these limitations. The first strategy is the hydrophobization of sugar through different methods like boronic acid or production of acetals (Sarney and Vulfson, 1995). The second strategy selects the appropriate solvent or solvents to solubilize the carbohydrate and the acylating agent and where the enzyme is active, like in the previously mentioned works. In the third strategy the right enzyme and support is chosen. The optimization of solvent/enzyme/support is part of this invention.

Practical purification of these compounds is scarcely described in literature. In bench scale, flash chromatography is useful (Baker et al., 2000), however like preparative chromatography (Jaspers et al., 1987) is complicated and expensive at an industrial level Most patents describe purification methods using solvents (Schaefer, 2005), or a two-step process: precipitation followed by an alcohol wash (de-la-Motte et al., 1991). None of these methods was useful for the purification of our bioconjugates, so the purification method proposed is new.

The application of the products generated by this invention come from the biological and techno functional activities they present. Literature and patents of bioconjugates formed by simple sugars and fatty acids mainly describe techno functional properties such as emulsifiers and food surfactants; cosmetic applications like capillary treatments, eyelashes treatments and deodorants; and antimicrobial effect, probiotic properties when FOS are used, antitumoral and pharmaceutical vector. Since bioconjugates with branched fructans like the ones of *A. tequilana* have not been synthetized, these properties have not been studied in these new molecules and they might not have the same properties of simple sugar or lineal FOS bioconjugates. So the biological and techno- functional activities of the bioconjugates and their uses were tested. Finally the anti-inflammatory activity had not been previously reported for this type of bioconjugates.

### BRIEF DESCRIPTION OF THE DRAWINGS

The present invention regards the synthesis of bioconjugated molecules formed between two or more of the following functional groups: prebiotics, triglycerides, fatty acids, sugars, anti-inflammatories; with its production process (synthesis and purification); and its applications as prebiotic nutraceutical, anti-inflammatory, antitumoral, intestinal vector, techno functional ingredient for food applications as emulsifier and fat substitute, and cosmetic; which are possible since these are non toxic molecules. The characteristic details of these molecules and the production process are clearly shown in the following description and Figures, which are mentioned as examples and should not be considered to limit the scope of the present invention:
Figure 1. General process for producing and obtaining bioconjugates;
Figure 2. Optional drying process for trace water and solvent elimination of the bioconjugates;
Figure 3. Optional purification process with diluted alkali for the elimination of acylating molecules form the bioconjugates;
Figure 4. Optional purification process with hot water for the separation of water soluble bioconjugates;
Figure 5. Optional purification process with water at room temperature for the separation of water soluble bioconjugates;
Figure 6. Optional drying process of bioconjugates with acylating traces;
Figure 7. Optional drying process of bioconjugates in aqueous solution;
Figure 8. HPLC chromatogram of bioconjugates synthetized using examples 1 (A) and 2(B);
Figure 9. Toxicological evaluation of bioconjugates;
Figure 10. Prebiotic effect of the new bioconjugates molecules.
Figure 11. Anti-inflammatory activity of the new bioconjugated molecules (A) *in vitro,* (B) *in vivo;*
Figure 12. Antitumoral activity of the new bioconjugated molecules;
Figure 13. Hydrolysis kinetic of the new bioconjugated molecules in an *ex vivo* digestive tract simulator; and
Figure 14. Examples of the techno functional application in food of the new bioconjugated molecules: (A) as emulsifier, (B) as fat substitute.

### BEST KNOWN METHOD FOR THE INVENTION

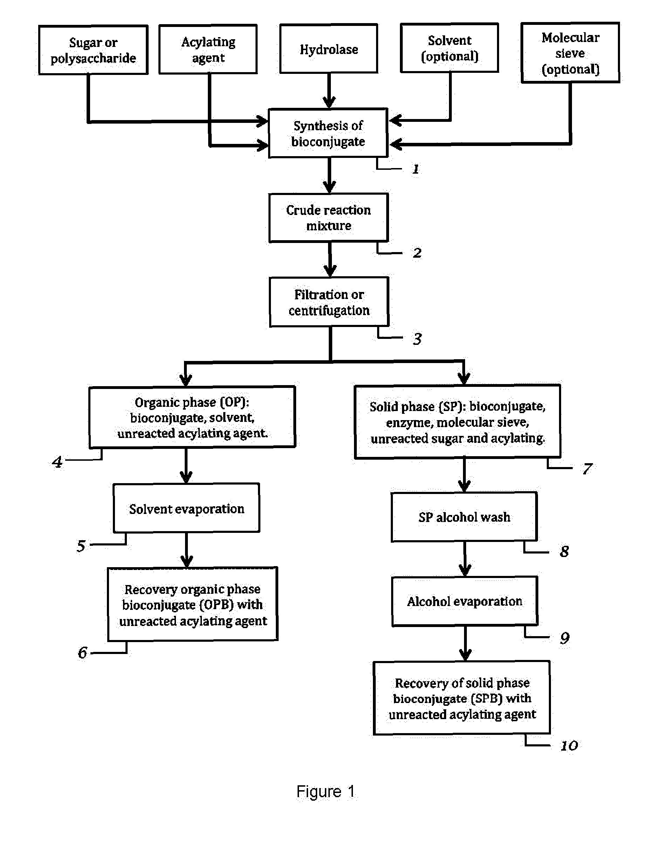

The process of the present invention is shown in Figures 1 to 4. In Figure 1 sugars, oligosaccharides or polysaccharides are FOS, GOS or other branched oligosaccharides with β(2→1) and β(2→6) links, such as agave fructans, raw or purified; when purified oligosaccharides with short (DP<10) or long (DP>10) degree of polymerization can be used. As it is a mixture of sugars, not a single bioconjugate is obtained but a mixture of bioconjugated molecules depending on the sugar used as substrate and the acylating agent.

The acylating agent can be a a) carboxylic acid (free fatty acid) of medium chain (8-12 carbons) or long chain (>12 carbons); or b) one of its esters (methyl, ethyl, vinyl, etc.); or c) an oil with different carboxylic acids, saturated or unsaturated such as omega-3; or d) a mixture of esters oils such as commercially available ethyl esters of omega-3. When the acylating agents are mixtures of fatty acids or their esters the complexity of the produced molecules increases.

The enzyme used in this invention is part of the serine-hydrolases, such as proteases, lipases, esterases, cutinases or any other that act or synthetizes an ester link. For better productivity the enzyme can be immobilized making its recovery and reuse easier. If the bioconjugated molecules will be used in foods, the enzyme must be food grade, if they will be used in pharmaceuticals or cosmetics, the enzyme should comply with the required standards.

Regarding solvent, the reaction can be carried out in organic solvents, including hydrophobic solvents such as hexane, heptane, isooctane, decane, etc.; hydrophilic solvents such as 2-methyl-2propanol, 2-metyl-2-butanol, acetone, etc.; as well as biphasic systems with hydrophobic solvents/water; or in the absence of solvents, being the acylating agent the solvent. The molecular sieve (porous silica, zeolite or clay with pore size of 3-4 A, or any other water absorbent) is optional when the acylating agent produces water as reaction sub-product.

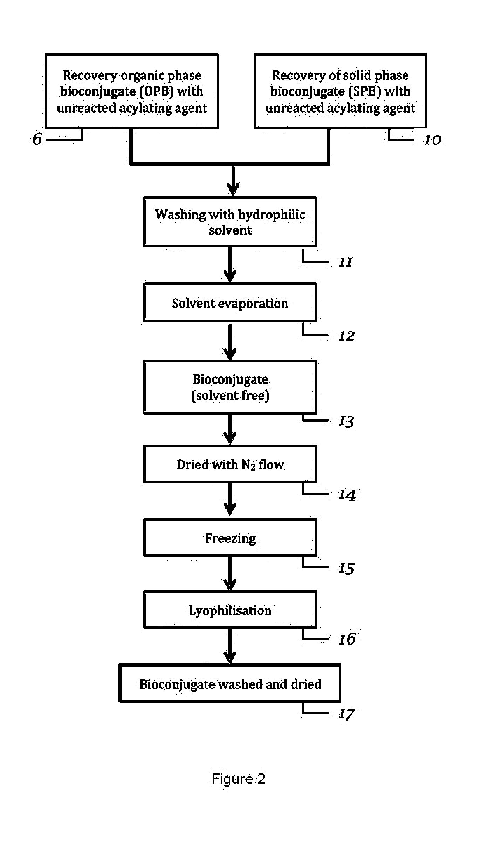

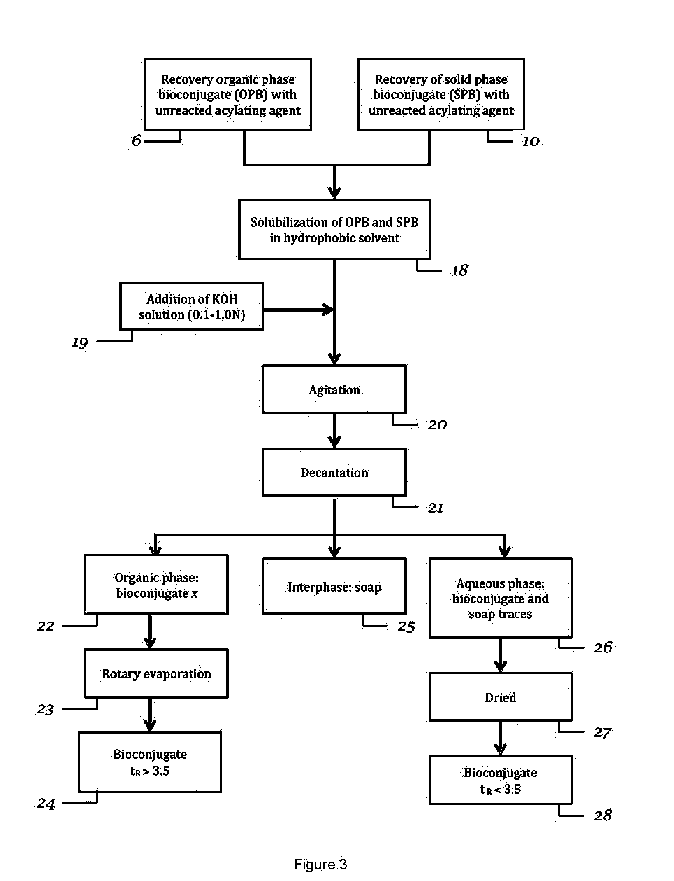

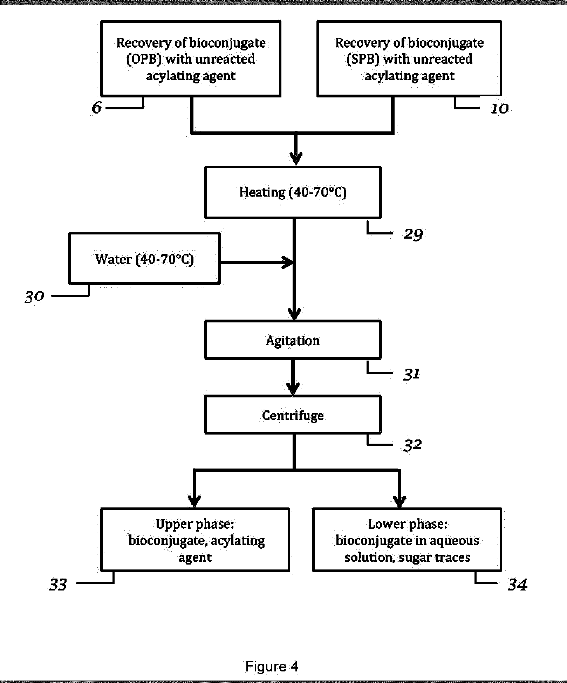

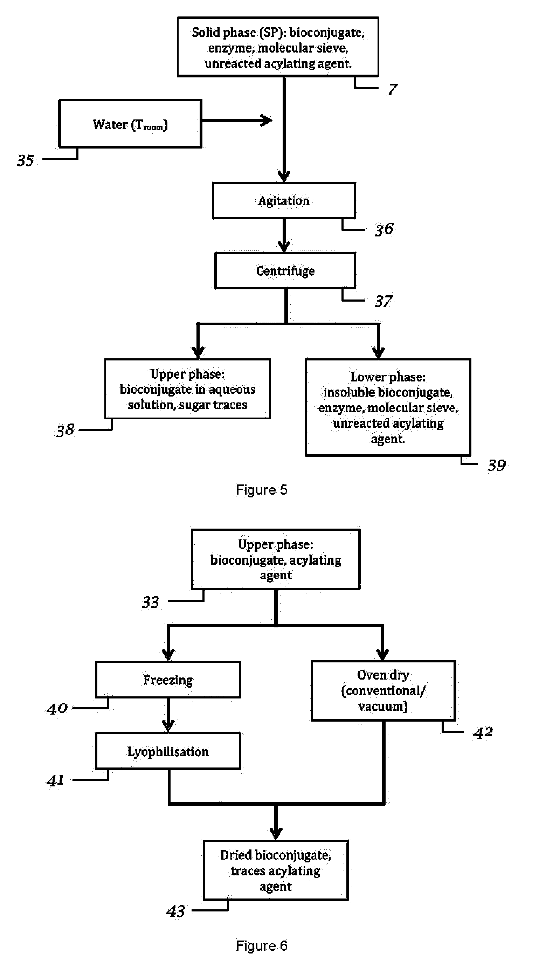

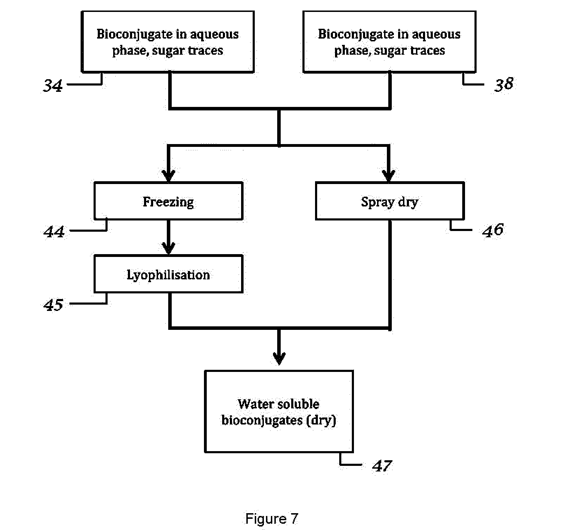

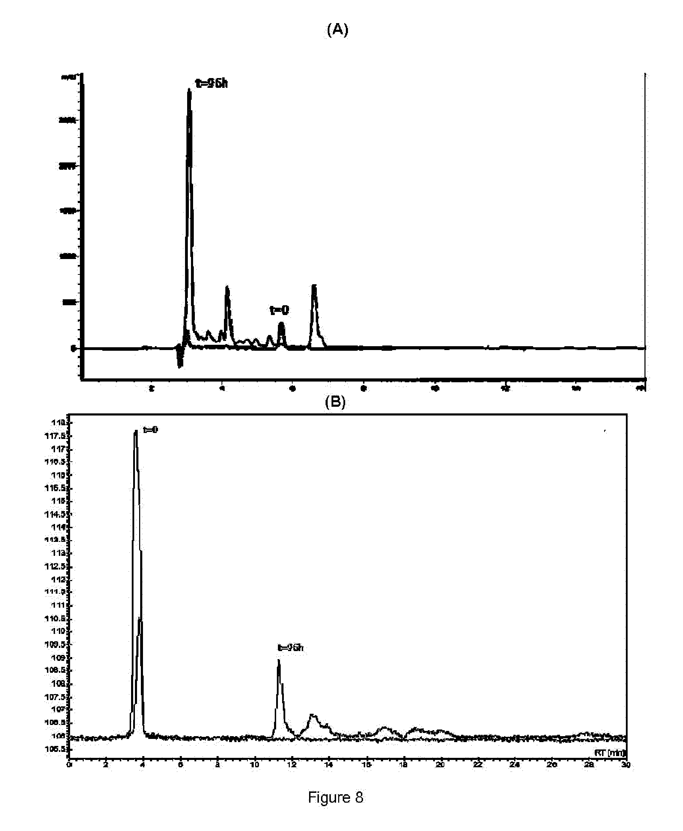

The synthesis process of a mixture of bioconjugated molecules, also denoted as "bioconjugates" in the present invention, includes the following steps:
*1. Synthesis reaction.* In this step sugars, oligo- or polysaccharides, are conjugated (esterified) with the acylating agent in a ratio of 1:1 to 1:10 w/w, in a reaction biocatalyzed by the enzyme which is added in a ratio of 1:1 to 1:10 w/w regarding the acylating agent; in a solvent with a ratio of 1:2 to 1:100 w/v regarding the acylating agent; in an hermetically sealed container, heated to a temperature that ensures the acylating agent is in liquid state when is a solvent free reaction or at a temperature below the boiling point of the solvent (generally between 40 and 80 °C), under agitation (manual, mechanic, magnetic, orbital, vibrations, thermic or passive diffusion) that allows good mass transfer (100-1000 r.p.m.). Optionally molecular sieve or other absorbent is added, when the acylating agent produces water during the reaction, in a ratio of 1:1 to 1:5 w/w. The reaction time is between 48 and 120h. At the end of the reaction the mixture is called "crude reaction mixture" (2) and is composed by the bioconjugates, unreacted acylating agent and sugars, and the enzyme (also the molecular sieve it was added).
2. *Filtering.* As observed in Figure 1, in the process of this invention, unlike other previously described inventions, the bioconjugates are also recovered from the solid phase (***10***) besides the liquid phase (***6***), by filtration or centrifugation (***3***), using Whatman filters number 1, 3, 4, or similar, and/or by centrifugation. From this step the liquid "organic phase" (OP, ***4***) contains the bioconjugates, solvent and the unreacted acylating agent, and in the retained "solid phase" (SP, ***7***), the bioconjugate, enzyme, molecular sieves (if added), unreacted sugars and acylating agent.
3. Bioconjugate recovery from "organic phase" is carried out by solvent evaporation (omitted if the acylating agent was the solvent). This is achieved by heating above the boiling point of the solvent used or using a rotary evaporator with reduced pressure and appropriate temperature. The dried product is called "organic phase bioconjugate" (OPB, ***6***) and although it might have unreacted acylating agent, they can be used in the applications described.
4. Bioconjugate recovery from "solid phase" is carried out by washing with an alcohol, such as methanol, ethanol, *t*-butanol, isopropanol or other hydrophilic solvent, in a ratio of 1:2 to 1:5 w/v. In the solid phase of this step (***8***), the enzyme, molecular sieve (if added) and unreacted sugars are recovered; while in the liquid phase, after alcohol evaporation as described in step 3, the "solid phase bioconjugates" (SPB, ***10***) are recovered, and although it might have unreacted acylating agent, they can be used in the applications described
5. Drying (optional). Figure 2 shows the optional process to completely drying the bioconjugates. The OPB and SPB are washed with a hydrophilic solvent such as an alcohol or acetone. Washing (***11***) removes the residual reaction solvent. The washing solvent is removed as described in step 3, so it can be recovered and recycled (***12***)**.** The solvent free bioconjugates (***13***) can be optionally dried using nitrogen when the reaction solvent is retained in the bioconjugates for complete elimination of the solvents and recovery of the bioconjugates (***14***). However once the organic solvent is removed, the bioconjugates can contain water due to the hydrophobicity of the sugar component in the bioconjugate, therefore they can be frozen (***15***) at a temperature of -5 to -80 °C and lyophilized (*16*) to remove residual water and recovery of washed and dried bioconjugates (*17*).
*6. Purification (optional):* If the reaction did not reached 100% yield or if the acylating agent was in excess, the acylating agent can be removed by several purification methods that will be described:
   a. With diluted alkali (Figure 3). The SPB (***10***) or OPB (***6***) are dissolved in a hydrophobic solvent in a ratio of 1:1 to 1:10 w/v (this is not necessary if the reaction was carried out in an hydrophobic solvent for OPB). An aqueous solution of diluted alkali (***19***), in a concentration of 0.1 to 1 N, is added to the solution *18* in a ratio of 1:1 to 1:5 v/v. After agitation (***20***) (manual, mechanic, magnetic, orbital, by vibration, thermic or passive diffusion), is decanted (***21***) and three phases separated; 1) organic phase (***22***) with bioconjugates with retention times larger than 3.5 min (***24***) (Figure 8-A) and can be evaporated following step 3 (***23***) for solvent recovery; 2) the interphase (***25***) containing soap and 3) an aqueous phase (***26***) containing the bioconjugates with retention times smaller than 3.5 min (Figure 8-A). The aqueous phase bioconjugates (***26***) are dried with an air flow or oven (***27***) to obtain bioconjugates (***28***) with retention times smaller than 3.5 min (Figure 8-A).
   b. With water: It can be hot (Figure 4) or room temperature water (Figure 5). With this purification method the bioconjugated molecules soluble in water can be separated. The hot water procedure is: the OPB (***6***) or SPB (***10***) are heated to 40 to 70 °C (***29***) and are washed with water at a temperature of 40 to 70 °C (***30***). The mixture is agitated manually or mechanically (***31***) and centrifuged at 1000-10000 r.p.m. (***32***). The upper phase (***33***) has the bioconjugates with unreacted acylating agent (if it was the case) and in the inferior phase the bioconjugate in aqueous phase with sugar traces (***34***). Alternatively the water-soluble product can be obtained from the reaction solid phase (SP, ***7***) washing with water at room temperature (***35***). The mixture is manually or mechanically agitated (***36***) and centrifuged at 1000-10000 r.p.m. (***37***). The upper phase (***38***) has the bioconjugate in solution with traces of unreacted sugar (if it was the case), while the lower phase (***39***) has a non water soluble bioconjugate with the enzyme, molecular sieve (if is was added) and the unreacted acylating agent (of it was the case). The upper phase bioconjugate with unreacted acylating agent (***39***) can be dried through freezing *(**40**)* and lyophilization *(**40**)* or oven drying (***42***), obtaining a non water soluble dried bioconjugate (***43***) with traces of acylating agent (if it was the case). The water soluble bioconjugates (***34*** or ***38***), with or without sugar traces, can be directly dried from the aqueous solution (Figure 7) through freezing (***44***), followed by lyophilization (***45***) or spray dry (***46***) to obtain dried water soluble bioconjugates (***47***).

Depending on the purification method employed the final state of the product

can be a water-soluble gel or a powder of molecules with longer retention times. The present invention has human and veterinarian applications.

### EXAMPLES OF APPLICATIONS

### Example 1. Bioconjugated molecules agave FOS + lauric acid.

16 g, agave FOS
50 g, vinyl laurate
500 ml, hexane
50 g, immobilized lipase (*C*. *antarctica* B)
50 g, molecular sieve of 3 A

The mixture is agitated at 60 °C for 96h, is filtrated and purified following one of the methods mentioned in step 6. The chromatogram of the bioconjugates synthetized this way is presented in Figure 8-A.

### Example 2. Bioconjugated molecules agave FOS + omega-3.

16 g, agave FOS
50 g, fish oil
500 ml, hexane
50 g, immobilized lipase (*C*. *antarctica* B)
50 g, molecular sieve of 3 A

The mixture is agitated at 60 °C for 96h, is filtrated and purified following one of the methods mentioned in step 6. The chromatogram of the bioconjugates synthetized this way is presented in Figure 8-B.

### Example 3. Application of bioconjugates in foods and pharmaceuticals.

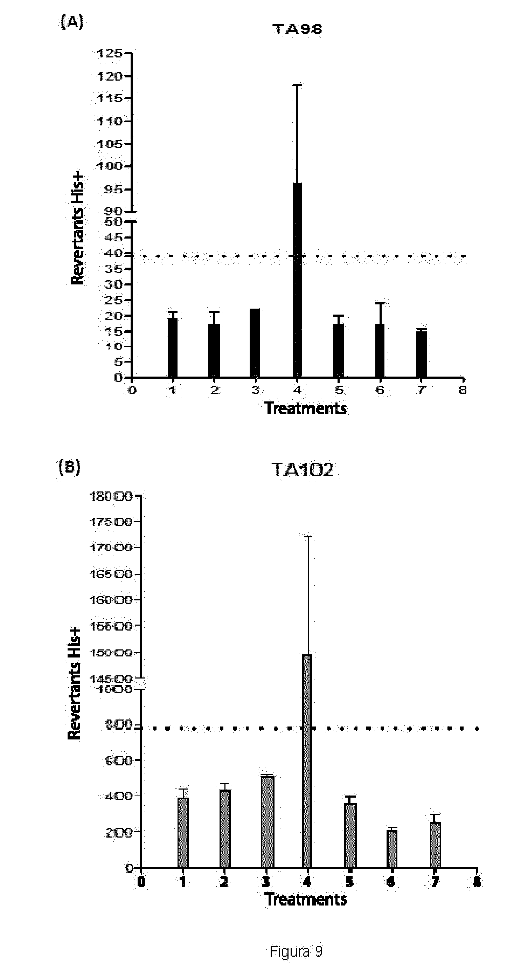

The bioconjugated molecules synthetized according to example 1 and purified by one of the methods in Figures 2 and 3, were evaluated for toxicity to confirm their applicability in foods and pharmaceuticals. Figure 9 shows the absence of toxicity in the two methods testes: (A) evaluation of mutagenicity in the TA98 strain by the generation of alkylating compounds; (B) evaluation of mutagenicity in the TA102 strain by the generation of oxidizing compounds. Both according to the method of Marron and Ames (Marron and Ames, 1983), that has been postulated as an acceptable test to detect as non-mutagenic the non- carcinogenic compounds (Dobo et al., 2006). The treatments were: (A): 1-Espontaneous reversion, 2-DMSO (solvent), 3-TWEN (solvent), 4-Picrolonic acid, 5-Control1, 6-Contorl2, 7-Bioconjugates. (B): 1-Espontaneous reversion, 2-DMSO (solvent), 3-TWEN (solvent), 4-4-nitroquinalone, 5-Control1, 6-Contorl2, 7-Bioconjugates.

### Example 4. Application of bioconjugates as prebiotics.

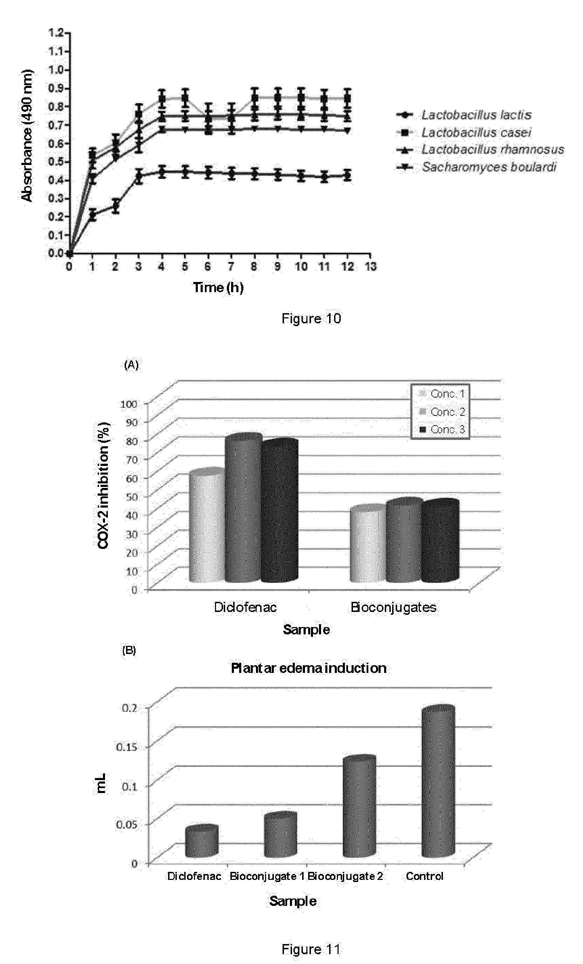

The bioconjugated molecules synthetized according to example 1 (purified by one of the methods in Figures 2 to 7), were used as carbon source for the growth of probiotic microorganisms, that showed acceptance in the consumption of the bioconjugates (figure 10). *Lactobacillus casei* and *L. rhamnosus* had the highest growth rates without statistical differences, concluding that the bioconjugates of branched oligosaccharides have prebiotic functions.

### Example 5. Application of bioconjugates as prebiotics anti-inflammatories.

The bioconjugated molecules synthetized according to example 1 (purified by one of the methods in Figures 2 to 7), can be used as anti-inflammatories as shown in Figure 11. Figure 11-A shows the *in-vitro* anti-inflammatory activity measured as COX-2 inhibition using a previously reported (Szymczak et al., 2008). The bioconjugates are compared to a known anti-inflammatory Diclofenac. Concentrations 1, 2 and 3 for Diclofenac were 50, 100 y 200 µg/ml respectively; for the bioconjugates were 400, 800 y 1600 µg/ml. (Mean values are presented for duplicates. Mean standard deviation was of 4.5%.
Figure 11-B shows the *in-vivo* anti-inflammatory activity of the bioconjugates measured using the plantar edema induction methodology (Xu et al., 2012). Samples were evaluated at a dosis of 100 mg/kg. Results show the plantar edema induction of the animals two hours after inflammation induction with carrageenan. The smaller the size of edema translates as a higher anti-inflammatory activity. In this Figure "Bioconjugates 1" were purified according to the method in Figure 2 and "Bioconjugates 2" were purified according to the method in Figure 3.

For the concentrations tested, Figure 11-A shows that the bioconjugates have between 60% and 70% of the *in vitro* anti-inflammatory activity found for Diclofenac, without presenting its secondary and hepatotoxic effects (Aithal, 2011). Figure 11-B shows the *in vivo* anti-inflammatory activity. Bioconjugates 1 had an anti-inflammatory activity similar to Diclofenac while Bioconjugates 2 presented a lower activity.

### Example 6. Application of bioconjugates as antitumorals.

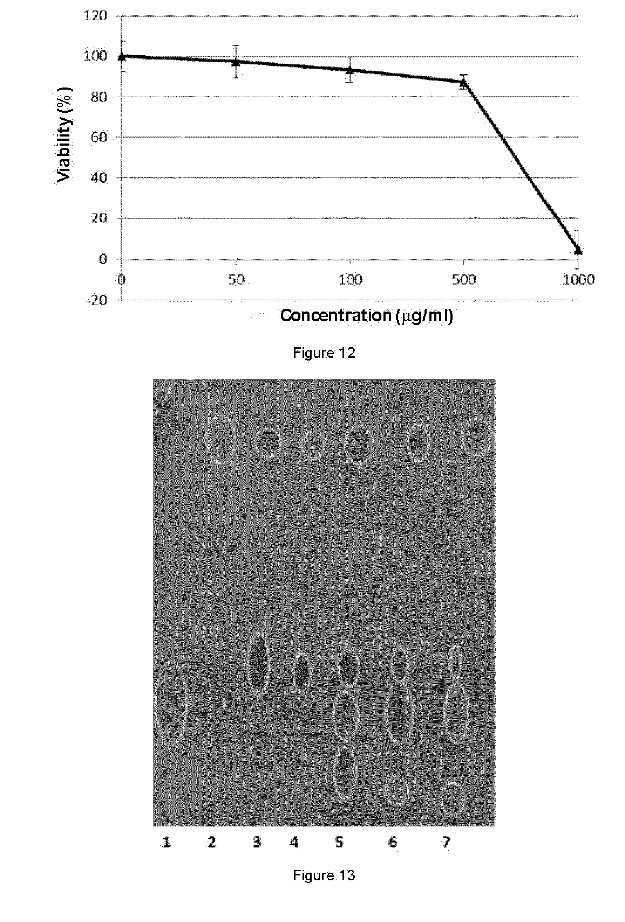

The bioconjugates synthetized according to example 1, purified according to the method in Figure 3, were used as cytotoxic agents in the HeLa cervical cancer cell line. As show in Figure 12, the bioconjugates are cytotoxic from a concentration of 500 µg/ml.

### Exampe 7. Application of bioconjugates as intestinal vector.

The bioconjugates synthetized according to example 1, purified according to one of the methods in step, were used as intestinal vector. In this case the prebiotic non-digestible part of the bioconjugates vectorizes the acylating part of the molecules. For this it was verified that the bioconjugates were not hydrolyzed before reaching the intestine in an intestinal tract simulator. The simulator was described by González-Avila et al., (González-Avila et al., 2012). Figure 13 shows a thin layer plate with the samples taken from the digestive tract simulator: 1-Lauric acid (standard), 2-Bioconjugates (control), 3-Food with bioconjugates in stomach, 4- Food with bioconjugates in small intestine, 5-Food with bioconjugates in ascendant colon, 6- Food with bioconjugates in transverse colon, 7- Food with bioconjugates in descending colon. Since no presence of lauric acid was observed in stomach and small intestine, it is concluded that the bioconjugates work as vectors to carry molecules of interest to the colon.

### Example 8. Application of bioconjugates as emulsifiers.

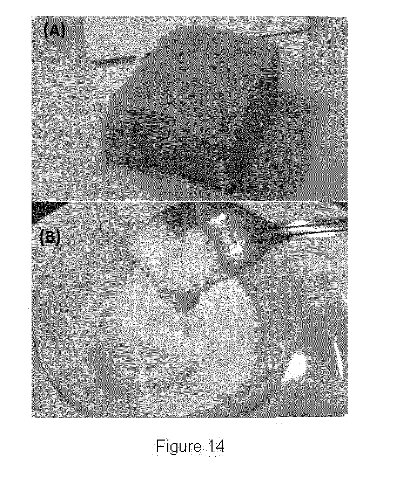

The bioconjugated molecules synthetized according to example 1 (purified by one of the methods in Figures 2 to 7), were used as emulsifiers in the preparation of a strawberry mousse (Figure 14-A), according to the following formula (%):

| | |
|---|---|
| Bioconjugates | 0.5 |
| Gelatin | 0.5 |
| Strawberry | 47.6 |
| Plain yogurt | 37.55 |
| Sour cream | 11.85 |
| Sugar | 4.0 |

### Example 9. Application of bioconjugates as fat substitute.

The bioconjugated molecules synthetized according to example 1 (purified by one of the methods in Figures 2 to 7), were used as fat substitute in the preparation of a coconut shake (Figure 14-B), according to the following formula (%):

| | |
|---|---|
| Bioconjugates | 0.43 |
| Gelatin | 0.43 |
| Evaporated milk | 31.56 |
| Condensed milk | 24.5 |
| Coconut- vanilla flavor | 0.16 |
| Hot water | 10.22 |

### Example 10. Cosmetic formulation using bioconjugates.

The bioconjugated molecules synthetized according to examples 1 or 2 (purified by one of the methods in Figures 2 to 7), were used in the preparation of a cosmetic emollient, according to the following formula (%):

| | |
|---|---|
| Bioconjugates | 6.0 |
| Liquid silicone f350 cts | 10.0 |
| Sorbitan | 5.0 |
| Stearic acid | 4.0 |
| Mineral oil | 2.0 |
| Lanoline | 1.0 |
| Cetyl alcohol | 1.0 |
| Triethanolamine | 0.9 |
| Antioxidant | 0.1 |
| Purified water c.s.p. | 100 |

## Claims

1. Bioconjugated molecules with biological and techno-functional activities with a weight ratio of 1:1 to 1:10 sugars, oligosaccharides or branched polysaccharides: carboxylic acid or its derivative.

2. Bioconjugated molecules with biological and techno functional activities according to claim 1, **characterized by** its sugar/oligosaccharide/polysaccharide component, which is one of the following:
a. FOS GOS or other branched oligosaccharides with β(2→1) and β(2→6) links, such as crude agave fructans;
b. FOS GOS or other branched oligosaccharides purified (mineral and color free); or
c. FOS GOS or other branched oligosaccharides separated by chain size smaller or larger DP 10; and
d. FOS GOS or other synthetic branched oligosaccharides.

3. Bioconjugated molecules with biological and techno-functional activities according to claim 1, **characterized by** its the carboxylic acids, which are chosen from the following ones:
i. free fatty acids
ii. fatty acid esters
iii. vegetable, animal or microbial oils.
iv. oils esters with a mixture of sizes and types of fatty acids.
v. commercial omega-3 concentrates.
vi. commercial fatty acid concentrates.

4. A process for the production of bioconjugated molecules with biological and techno-functional activities according to claims 1-3 including:
a. *reaction production*: mixing on a hermetically closed recipient with agitation at 100-1000 r.p.m. and incubation at 40-80 °C, two substrates according to claim 1 in a proportion of 1:1 to 1:10 w/w, in presence of an enzyme in a proportion of 1:1 to 1:10 w/w to the acylating agent; with or without solvent that if it is added is in a ratio of 1:2 to 1:100 w/v;
*b. filter* with Whatman filters numbers 1, 3, 4, or similar, or centrifuge for separation of the organic solvent and the immobilized enzyme.
*c*. *bioconjugates recovery from the "organic phase"* by solvent evaporation at reduced pressure (vacuum) in a rotary evaporator, heating at a temperature where the solvent boils depending on the vacuum used, or by heating at a temperature above the solvent boiling point.
*d. bioconjugates recovery from the "solid phase"* by washing with a hydrophilic solvent in a ratio of 1:2 to 1:15 v/v and further evaporation as in step 4c.

5. The process for the production of bioconjugated molecules with biological and techno-functional activities according to claim 4 **characterized by** the fact that the enzyme from step 4a is a serine-hydrolase that could be immobilized, and is preferably a protease, lipase, esterase or cutinase.

6. The process for the production of bioconjugated molecules with biological and techno-functional activities according to claim 4 **characterized by** the fact that in step 4a molecular sieves, zeolites, clays or other water absorbents can be added in a ratio of 1:1 to 1:5 w/w to the acylating agent.

7. The process for the production of bioconjugated molecules with biological and techno-functional activities according to claim 4 **characterized by** a drying step comprised by a hydrophilic solvent wash in a ratio of 1:2 to 1:15 v/w can be added, followed by evaporation according to step 4c, with an optional drying under nitrogen, followed by freezing at -5 to -80 °C and lyophilization.

8. The process for the production of bioconjugated molecules with biological and techno-functional activities according to claim 4 **characterized by** a purification step, which can be added to eliminate unreacted sugars and acylating agent, and can be carried out using a diluted alkali in a concentration of 0.1 to 1 N, or water in a ratio of 1:1 to 1:20 v/v with respect to the organic phase; recovering the purified bioconjugates in the interphase, organic phase and aqueous phase respectively, after drying according to step 4c or the process from claim 7.

9. The process for the production of bioconjugated molecules with biological and techno-functional activities according to claim 4 **characterized by** the agitation step, which can be manual, mechanic, magnetic, orbital, by vibrations, thermic or passive diffusion.

10. Bioconjugated molecules with biological and techno-functional activities **characterized by** its production from the process described in claim 4 or the variants of the process according to claims 5 to 9.

11. The bioconjugated molecules with biological and techno-functional activities according to claims 1, 2, 3 and 10 **characterized by** the absence of toxicity.

12. The bioconjugated molecules with biological and techno functional activities according to claims 1, 2, 3 and 10 **characterized by** having prebiotic activity.

13. The bioconjugated molecules with biological and techno functional activities according to claims 1, 2, 3 and 10 **characterized by** having anti-inflammatory activity.

14. The bioconjugated molecules with biological and techno functional activities according to claims 1, 2, 3 and 10 **characterized by** having antitumoral activity.

15. The bioconjugated molecules with biological and techno functional activities according to claims 1, 2, 3 and 10 characterized because they can be used as intestinal vectors.

16. The bioconjugated molecules with biological and techno functional activities according to claims 1, 2, 3 and 10 characterized because they can be used as emulsifiers.

17. The bioconjugated molecules with biological and techno functional activities according to claims 1, 2, 3 and 10 characterized because they can be used as fat substitutes.

18. The bioconjugated molecules with biological and techno-functional activities according to claims 1, 2, 3 and 10 characterized because they can be used as cosmetic emollients.

19. The bioconjugated molecules with biological and techno-functional activities according to claims 1, 2, 3 and 10 -18 characterized because depending on the purification method they can be powder, gel or cream.

20. The application of bioconjugated molecules with biological and techno-functional activities according to claims 1 to 19 as active ingredients in food, nutraceutics, cosmetics and pharmaceuticals.
